(19) Europäisches Patentamt
European Patent Office
Office européen des brevets

(11) **EP 3 598 455 A1**

(12) **EUROPEAN PATENT APPLICATION**

(43) Date of publication:
**22.01.2020 Bulletin 2020/04**

(51) Int Cl.:
**G16H 20/30** *(2018.01)*

(21) Application number: **18184602.3**

(22) Date of filing: **20.07.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
 • **BEREZHNYY, Igor**
 **5656 AE Eindhoven (NL)**

 • **VAN EE, Raymond**
 **5656 AE Eindhoven (NL)**
 • **AARTS, Ronaldus Maria**
 **5656 AE Eindhoven (NL)**
 • **JOHNSON, Mark Thomas**
 **5656 AE Eindhoven (NL)**
 • **VAN LIESHOUT, Ron Martinus Laurentius**
 **5656 AE Eindhoven (NL)**
 • **DELLIMORE, Kiran Hamilton J.**
 **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **A COMPUTER-IMPLEMENTED METHOD AND APPARATUS FOR DETERMINING A TARGET ACTIVITY RATE FOR A SUBJECT**

(57) According to an aspect, there is provided a computer-implemented method of determining a target activity rate for a subject, the method comprising determining a first activity that is being performed by the subject or that is to be performed by the subject, wherein performing the first activity comprises repeating a first activity cycle; determining an energy amount to be exerted by the subject in performing each first activity cycle; determining a target activity rate at which the subject should repeat the first activity cycle such that the heart rate of the subject is in a target range, wherein the target activity rate is determined based on the determined energy amount and a first criterion relating to the target range; and outputting an indication to the subject relating to the determined target activity rate.

Fig. 1

**Description**

FIELD OF THE INVENTION

This disclosure relates to a computer-implemented method and apparatus for determining a target activity rate for a subject.

BACKGROUND OF THE INVENTION

For people with heart failure (HF), to slow progression of the illness and support a healthy lifestyle, it is necessary to maintain a certain level of physical activity by performing exercises. However, exercises need to be conducted with caution, and particularly at all times heart rate (HR) has to be kept within safe limits defined by a doctor or other care provider. Typically this means that heart rate should be monitored during exercise, for example with a heart rate monitor. However, arrhythmia (i.e. irregular heartbeats) is very common among people with heart failure (estimated to be between 30-90% of people with heart failure), and so in many cases the arrhythmia will lead to unreliable heart rate measurements.

Many people with heart failure lack confidence in performing therapeutic training exercises or physically strenuous activities, which causes them to feel out of breath or elevate their heart rate since they fear either death or experiencing further heart failure. As a result, they tend to avoid doing these activities, which in turn leads to a deterioration in their physical condition, thereby increasing their risk of a myocardial infarction (MI) or the (further) development of heart failure.

One approach to monitoring and limiting heart rate is to monitor energy expenditure. Whilst it is possible to monitor the energy expenditure of people in a laboratory setting (for example while wearing an energy expenditure meter, such as a mask that measures exhaled carbon dioxide ($CO_2$)), it is not practical to apply this outside of the laboratory.

Therefore, there is a need for improved ways of monitoring and advising a subject, particularly a subject that has arrhythmia for whom direct heart rate monitoring does not provide reliable measurements.

SUMMARY OF THE INVENTION

As noted above, due to the high occurrence of arrhythmias in people with heart failure, conventional heart rate 'fencing' approaches (i.e. putting an upper limit on the heart rate of the person) utilising, for example, wearable or exercise machine based heart rate monitors, are unreliable and potentially dangerous for people with heart failure.

In order to comply with current clinical practice (i.e. where an upper limit is placed on heart rate) it is foreseen that it will be required to translate energy into a heart rate, and/or a potential heart rate, which would occur if the person did not have arrhythmia. For moderate exercises (such as those that might be undertaken by people with heart failure), heart rate increases generally linearly with the power exerted by the person.

The techniques described herein provide an alternative to direct fencing of heart rate (i.e. measuring heart rate directly, and comparing that heart rate to a threshold) that effectively achieves the same result, and is also more applicable or more reliable for people with arrhythmia (including people with both heart failure and arrhythmia). In particular the techniques enable people to stay within a safe zone of physical load, and provide the ability to foresee whether they will exceed the limit. Generally, the techniques provide that the activity of the person is measured, the activity is linked with an energy expenditure by the person, and this energy expenditure is used as a surrogate heart rate metric. This surrogate heart rate metric can be used for both fencing (i.e. enforcing an upper limit on the physical exertion of the person) and pacing the intensity of the exercise, as the progression of the surrogate heart rate metric over time with continued or increasing activity can be predicted in the short term, and this prediction used to provide feedback to the person to maintain or slow down the pace in order to stay within a safe physical exertion boundary.

According to a first specific aspect, there is provided a computer-implemented method of determining a target activity rate for a subject, the method comprising determining a first activity that is being performed by the subject or that is to be performed by the subject, wherein performing the first activity comprises repeating a first activity cycle; determining an energy amount to be exerted by the subject in performing each first activity cycle; determining a target activity rate at which the subject should repeat the first activity cycle such that the heart rate of the subject is in a target range, wherein the target activity rate is determined based on the determined energy amount and a first criterion relating to the target range; and outputting an indication to the subject relating to the determined target activity rate. Thus, this aspect provides that the subject can be advised of a suitable target activity rate for performing an activity cycle that will enable them to keep their heart rate within the target range. This aspect is particularly suitable to those subjects for whom direct heart rate monitoring does not provide a reliable indication of their heart rate or exertion level (e.g. subjects with arrhythmia).

In some embodiments, the step of determining the first activity comprises one of (i) receiving an input from the subject or other user identifying an activity that is being performed by the subject or that is to be performed by the subject;

(ii) determining the activity from an exercise programme for the subject; and (iii) receiving measurements of the movements of the subject from one or more sensors and processing the received measurements to determine the activity that is being performed by the subject. Thus, the activity can be determined automatically, or determined based on an input.

In some embodiments, the step of determining the energy amount comprises retrieving information on the energy amount to be exerted in performing each first activity cycle from a database. In these embodiments, the retrieved information can be specific to the subject. In these embodiments, the method can further comprise the steps of measuring the exhaled carbon dioxide, $CO_2$, from the subject while the subject is performing the first activity; measuring movements of the subject while the subject is performing the first activity; processing the exhaled $CO_2$ measurements and the movement measurements to determine the amount of exhaled $CO_2$ from the subject for each first activity cycle; determining the energy amount exerted by the subject for each first activity cycle from the determined exhaled $CO_2$ for each first activity cycle; and storing the determined energy amount exerted by the subject for each first activity cycle in the database. In alternative embodiments, the retrieved information relates to a population of subjects.

In some embodiments, the first criterion represents the target range of the heart rate in terms of a target range of power to be exerted by the subject. In these embodiments, the step of determining the target activity rate can comprise determining the target activity rate from a power in the target range of power divided by the determined energy amount exerted by the subject for each first activity cycle. In these embodiments, the target activity rate can be a maximum target activity rate and the step of determining the target activity rate can comprise determining the maximum target activity rate from a maximum power in the target range of power divided by the determined energy amount exerted by the subject for each first activity cycle. This embodiment has the advantage that the subject can be advised of the highest permitted rate of repetition.

In some embodiments, the output indication is one or more of the determined target activity rate and a timing indicator corresponding to repetitions of the first activity cycle. The timing indicator can be useful to inform the subject when or how often each activity cycle can be repeated.

In some embodiments, the method further comprises the steps of measuring the heart rate of the subject over time; analysing the measurements of the heart rate to identify changes in a resting heart rate for the subject over time; and adjusting the first criterion based on identified changes in the resting heart rate. In these embodiments, the step of adjusting the first criterion based on identified changes in the resting heart rate can comprise reducing a maximum heart rate of the target range for the heart rate if the resting heart rate increases over time; and adjusting the first criterion to the target range with the reduced maximum heart rate. These embodiments enable the target activity rate to be adapted based on changes in physical fitness of the subject.

In some embodiments, the method further comprises the steps of receiving one or more inputs relating to one or more parameters indicating a current status of the subject; and adjusting one or both of (i) the determined energy amount to be exerted by the subject in performing each first activity cycle, (ii) the first criterion based on the received one or more parameters. In these embodiments, the one or more parameters comprise any one or more of (i) a weight of the subject; (ii) a body composition of the subject; (iii) a proportion of oxygen in the air where the subject is located; (iv) environmental temperature and/or humidity; (v) medication for the subject; (vi) metabolic rate for the subject; and (vii) an occurrence of arrhythmia. These embodiments enable the target activity rate to be adapted to the current characteristics or situation of the subject.

In some embodiments, the method further comprises determining a first activity rate at which the subject is repeating the first activity cycle; comparing the first activity rate to the target activity rate; and outputting an indication to the subject based on the result of the comparison. The output indication can indicate to the subject whether they can safely increase the repetition rate, or indicate that they should decrease the repetition rate to remain in the same limit. In these embodiments, the step of determining the first activity rate comprises receiving measurements of the movements of the subject from one or more sensors; and processing the received measurements to determine the activity that is being performed by the subject and a rate at which activity cycles for the determined activity are being performed.

In some embodiments, the first activity and first activity cycle respectively comprise one of: (i) walking and one or more footsteps; (ii) running and one or more footsteps; (iii) going up or down stairs and going up or down one step; (iv) riding a bicycle and one turn of the pedals; and (v) physical exercise and one repetition of the exercise.

According to a second aspect, there is provided a computer program product comprising a computer readable medium having computer readable code embodied therein, the computer readable code being configured such that, on execution by a suitable computer or processor, the computer or processor is caused to perform the method according to the first aspect or any embodiment thereof.

According to a third specific aspect, there is provided an apparatus for determining a target activity rate for a subject, the apparatus comprising a processing unit configured to determine a first activity that is being performed by the subject or that is to be performed by the subject, wherein performing the first activity comprises repeating a first activity cycle; determine an energy amount to be exerted by the subject in performing each first activity cycle; determine a target activity rate at which the subject should repeat the first activity cycle such that the heart rate of the subject is in a target range, wherein the target activity rate is determined based on the determined energy amount and a first criterion relating to the target range; and output an indication to the subject relating to the determined target activity rate. Thus, this aspect provides that the subject can be advised of a suitable target activity rate for performing an activity cycle that will enable them to keep their heart rate within the target range. This aspect is particularly suitable to those subjects for whom direct heart rate monitoring does not provide a reliable indication of their heart rate or exertion level (i.e. subjects with arrhythmia).

In some embodiments, the processing unit is configured to determine the first activity by one of (i) receiving an input from the subject or other user identifying an activity that is being performed by the subject or that is to be performed by the subject; (ii) determining the activity from an exercise programme for the subject; and (iii) receiving measurements of the movements of the subject from one or more sensors and processing the received measurements to determine the activity that is being performed by the subject. Thus, the activity can be determined automatically, or determined based on an input.

In some embodiments, the processing unit is configured to determine the energy amount by retrieving information on the energy amount to be exerted in performing each first activity cycle from a database. In these embodiments, the retrieved information can be specific to the subject. In these embodiments, the processing unit can be further configured to measure the exhaled carbon dioxide, $CO_2$, from the subject while the subject is performing the first activity; measure movements of the subject while the subject is performing the first activity; process the exhaled $CO_2$ measurements and the movement measurements to determine the amount of exhaled $CO_2$ from the subject for each first activity cycle; determine the energy amount exerted by the subject for each first activity cycle from the determined exhaled $CO_2$ for each first activity cycle; and store the determined energy amount exerted by the subject for each first activity cycle in the database. In alternative embodiments, the retrieved information relates to a population of subjects.

In some embodiments, the first criterion represents the target range of the heart rate in terms of a target range of power to be exerted by the subject. In these embodiments, the processing unit can be configured to determine the target activity rate can by determining the target activity rate from a power in the target range of power divided by the determined energy amount exerted by the subject for each first activity cycle. In these embodiments, the target activity rate can be a maximum target activity rate and the processing unit can be configured to determine the target activity rate by determining the maximum target activity rate from a maximum power in the target range of power divided by the determined energy amount exerted by the subject for each first activity cycle. This embodiment has the advantage that the subject can be advised of the highest permitted rate of repetition.

In some embodiments, the output indication is one or more of the determined target activity rate and a timing indicator corresponding to repetitions of the first activity cycle. The timing indicator can be useful to inform the subject when or how often each activity cycle can be repeated.

In some embodiments, the processing unit is further configured to receive measurements of the heart rate of the subject over time; analyse the measurements of the heart rate to identify changes in a resting heart rate for the subject over time; and adjust the first criterion based on identified changes in the resting heart rate. In these embodiments, the processing unit can be configured to adjust the first criterion based on identified changes in the resting heart rate by reducing a maximum heart rate of the target range for the heart rate if the resting heart rate increases over time; and adjusting the first criterion to the target range with the reduced maximum heart rate. These embodiments enable the target activity rate to be adapted based on changes in physical fitness of the subject.

In some embodiments, the processing unit is further configured to receive one or more inputs relating to one or more parameters indicating a current status of the subject; and adjust one or both of (i) the determined energy amount to be exerted by the subject in performing each first activity cycle, (ii) the first criterion based on the received one or more parameters. In these embodiments, the one or more parameters comprise any one or more of (i) a weight of the subject; (ii) a body composition of the subject; (iii) a proportion of oxygen in the air where the subject is located; (iv) environmental temperature and/or humidity; (v) medication for the subject; (vi) metabolic rate for the subject; and (vii) an occurrence of arrhythmia. These embodiments enable the target activity rate to be adapted to the current characteristics or situation of the subject.

In some embodiments, the processing unit is further configured to determine a first activity rate at which the subject is repeating the first activity cycle; compare the first activity rate to the target activity rate; and output an indication to the subject based on the result of the comparison. The output indication can indicate to the subject whether they can safely increase the repetition rate, or indicate that they should decrease the repetition rate to remain in the same limit. In these embodiments, the processing unit is configured to determine the first activity rate by receiving measurements of the movements of the subject from one or more sensors; and processing the received measurements to determine the activity that is being performed by the subject and a rate at which activity cycles for the determined activity are being performed.

In some embodiments, the first activity and first activity cycle respectively comprise one of: (i) walking and one or more footsteps; (ii) running and one or more footsteps; (iii) going up or down stairs and going up or down one step; (iv) riding a bicycle and one turn of the pedals; and (v) physical exercise and one repetition of the exercise.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a block diagram illustrating an apparatus according to an exemplary embodiment;
Fig. 2 is a flow chart illustrating a method according to an exemplary embodiment; and
Fig. 3 is an illustration of power and energy expended during an activity that comprises activity cycles.

DETAILED DESCRIPTION OF EMBODIMENTS

As outlined above, in the range of moderate exercise recommended to people with heart failure, the heart rate is generally linearly related to the power exerted by the person, i.e. the power is proportional to the rate of movement. As an example the heart rate increases almost linearly with the speed of running (i.e. the heart rate increases almost linearly with step rate).

Therefore, with the aim of limiting the heart rate of the person when exercising (i.e. avoiding the heart rate exceeding an upper limit, e.g. a limit that is set by a physician), the techniques provided herein aim to limit the power generated by the person when exercising, so that the heart rate remains in the safe zone (i.e. below an upper limit). This approach avoids the requirement to monitor heart rate directly and compare it to a threshold, and so the approach is applicable to people with arrhythmia.

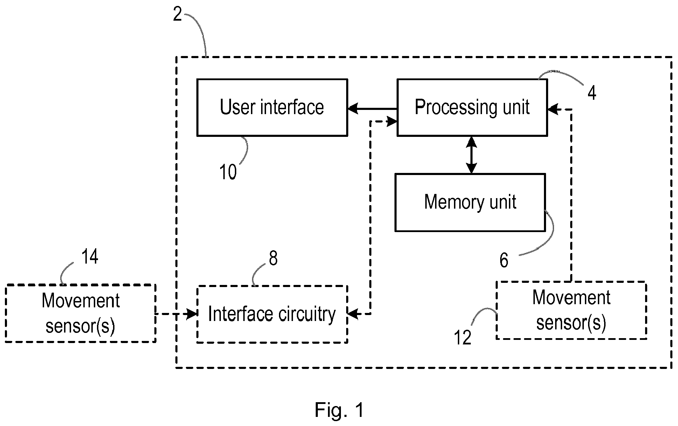

Fig. 1 is a block diagram of an apparatus 2 that can be used to implement the techniques described herein. The apparatus 2 may take any desired form, and in some embodiments, the apparatus 2 is in the form of, or part of, a device that can be worn or carried by a subject (the person to whom the techniques described herein are to be applied), for example a smart watch, a smart phone, a tablet computer, or any other type of portable electronic device. In other embodiments, the apparatus 2 can be in the form of, or part of, a laptop, a desktop computer, a television set-top box, a server, etc.

The apparatus 2 includes a processing unit 4 that controls the operation of the apparatus 2 and that can be configured to execute or perform the methods described herein. The processing unit 4 can be implemented in numerous ways, with software and/or hardware, to perform the various functions described herein. The processing unit 4 may comprise one or more microprocessors or digital signal processor (DSPs) that may be programmed using software or computer program code to perform the required functions and/or to control components of the processing unit 4 to effect the required functions. The processing unit 4 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 4 is connected to a memory unit 6 that can store data, information and/or signals for use by the processing unit 4 in controlling the operation of the apparatus 2 and/or in executing or performing the methods described herein. For instance, the memory unit 6 can store a database that includes information on an amount of energy that would be exerted by the subject in performing individual cycles of different physical activities (e.g. one walking footstep, one running footstep, climbing one stair, etc.), although in some embodiments this information can be stored in a memory unit or database that is separate from the apparatus 2. The memory unit 6 can also store information relating to a heart rate limit or target heart rate range for the subject, for example including at least an upper limit on the heart rate, and optionally also a lower heart rate, although again this information could be stored separately from the apparatus 2. The memory unit 6 may also or alternatively store information on a criterion that relates a target range for the heart rate (or an upper limit for the heart rate) to a range of power (or an upper limit on the power) exerted by the subject, although again this information could be stored separately from the apparatus 2.

In some implementations the memory unit 6 stores computer-readable code that can be executed by the processing unit 4 so that the processing unit 4 performs one or more functions, including the methods described herein. The memory unit 6 can comprise any type of non-transitory machine-readable medium, such as cache or system memory including volatile and non-volatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In some embodiments, the apparatus 2 also includes interface circuitry 8 for enabling a data connection to and/or data exchange with other devices, including any one or more of servers, databases, user devices, and sensors. The connection may be direct or indirect (e.g. via the Internet), and thus the interface circuitry 8 can enable a connection between the apparatus 2 and a network, such as the Internet, via any desirable wired or wireless communication protocol. For example, the interface circuitry 8 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 8 (and thus apparatus 2) may include one or more suitable antennas for transmitting/receiving over a transmission medium (e.g. the air). Alternatively, in the case of a wireless connection, the interface circuitry 8 may include means (e.g. a connector or plug) to enable the interface circuitry 8 to be connected to one or more suitable antennas external to the apparatus 2 for transmitting/receiving over a transmission medium (e.g. the air). The interface circuitry 8 is connected to the processing unit 4.

The interface circuitry 8 can be used to receive information on an activity that is being performed by the subject, and/or that is to be performed by the subject. The interface circuitry 8 can also or alternatively be used to receive measurements of the movements of the subject from one or more movement sensors. Alternatively or in addition, where information required for implementing the techniques described herein is stored in a memory unit or database that is separate from the apparatus 2, the information can be retrieved by the processing unit 4 from the separate memory unit or database via the interface circuitry 8.

The apparatus 2 also comprises a user interface 10 that includes one or more components that enables the apparatus 2 to output information or data to the subject, including, for example, an indication relating to the target heart rate determined according to the techniques described herein. Thus the user interface 10 can comprise any suitable output component(s), including but not limited to a display screen, one or more lights or light elements, one or more loudspeakers, a vibrating element, etc. In some embodiments, the user interface 10 also includes one or more components for enabling the subject to input information, data and/or commands into the apparatus 2. The user interface 10 can comprise any suitable input component(s), including but not limited to a keyboard, keypad, one or more buttons, switches or dials, a mouse, a track pad, a touchscreen, a stylus, a camera, a microphone, etc.

It will be appreciated that a practical implementation of an apparatus 2 may include additional components to those shown in Fig. 1. For example the apparatus 2 may also include a power supply, such as a battery, or components for enabling the apparatus 2 to be connected to a mains power supply.

As noted above, in some embodiments measurements from one or more movement sensors are required for performing the techniques described herein, or embodiments of the techniques described herein. Therefore, in some embodiments, the apparatus 2 further comprises one or more movement sensors 12 that monitor the movements of the subject and output measurements of the movements of the subject to the processing unit 4 for analysis and processing. In these embodiments, the apparatus 2 is typically in a form that can be carried or worn by the subject (e.g. the apparatus 2 can be a smart watch). In alternative embodiments, one or more movement sensors 14 can be provided that are separate from the apparatus 2 that monitor the movements of the subject and output measurements of the movements of the subject to the processing unit 4 via the interface circuitry 8. In these embodiments, the one or more sensors 14 can be carried or worn by the subject. In either embodiment, the one or more movement sensors 12, 14 can comprise any one or more of an accelerometer, a magnetometer, a satellite positioning system receiver (e.g. a GPS receiver, a GLONASS receiver, a Galileo positioning system receiver), a gyroscope, an inertial sensor, and an air pressure sensor (that can provide measurements indicative of the altitude of the subject or changes in height/altitude of the subject). It will be appreciated that in some embodiments the apparatus 2 can include one or more movement sensors 12 and there can be one or more movement sensors 14 that are separate from the apparatus 2.

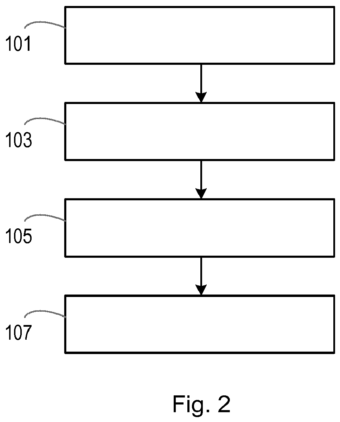

The flow chart in Fig. 2 illustrates an exemplary method according to the techniques described herein. One or more of the steps of the method can be performed by the processing unit 4 in the apparatus 2, in conjunction with any of the memory unit 6, interface circuitry 8 and user interface 10 as appropriate. The processing unit 4 may perform the one or more steps in response to executing computer program code, that can be stored on a computer readable medium, such as, for example, the memory unit 6.

In step 101, an activity (referred to herein as a 'first activity' or 'the activity') that is being performed by the subject or that is to be performed by the subject is determined. The activity comprises repeating an activity cycle (referred to herein as a 'first activity cycle' or 'the activity cycle'). As an example, the activity can be walking, and the relevant activity cycle can be one or more footsteps. As another example, the activity can be running, and the relevant activity cycle can be one or more footsteps. In another example, the activity can be going up or down stairs, and the relevant activity cycle can be going up or down one step. In yet another example, the activity can be riding a bicycle, and the relevant activity cycle can be one turn (e.g. one full turn) of the pedals. In yet another example, the activity can be a physical exercise (e.g. lifting weights, skipping, using a rowing machine, etc.), and the relevant activity cycle can be one repetition of the exercise.

The activity that is being performed by the subject or that is to be performed by the subject can be determined from an input provided to the apparatus 2 by the subject or by another user of the apparatus 2 (e.g. a care provider). This input can be a manual input via the user interface 10 (for example a selection of an activity presented on the user interface 10) or a signal from another electronic device via the interface circuitry 8. Alternatively, if the subject is performing the activity, the activity can be detected by analysing movement measurements from the movement sensor(s) 12, 14. As another alternative, the activity being performed or to be performed could be determined from an exercise programme for the subject. In any of these embodiments, the processing unit 4 can receive information indicative of the activity that is to be performed or that is being performed.

In step 103, the energy amount to be exerted by the subject in performing each activity cycle of the determined activity is determined. This step can comprise retrieving information on the energy amount to be exerted in performing each activity cycle from a database. The retrieved information may be specific to the subject. Alternatively, the retrieved information may relate to a population of subjects (e.g. a general population, or a population having a similar health status as the subject, etc.). In the case of a subject having heart failure, data for a population of subjects having HF may be used, which is weighted according to the severity of the HF (e.g. New York Heart Association (NYHA) class I, II, III etc.), the body-mass index (BMI) and/or the age of the HF subject. Where the information is specific to the subject, the information may have been derived from earlier measurements of the subject while the subject performed the first activity. For example, prior to step 101, the subject may have been required to perform the first activity and the carbon dioxide ($CO_2$) exhaled by the subject is measured during that activity (e.g. using a face mask and $CO_2$ sensor). The movements of the subject are also measured while the subject is performing the first activity. The movement measurements are processed to identify individual activity cycles, and the exhaled $CO_2$ measurements are processed to determine the amount of $CO_2$ exhaled by the subject for each activity cycle. The amount of $CO_2$ exhaled for each activity cycle is used to determine the energy amount exerted by the subject for each activity cycle, and this determined energy amount is stored in the database.

In step 105, a target activity rate at which the subject should repeat the activity cycle is determined so that the heart rate of the subject would be in a target range. The target activity rate for the repetition of the activity cycle is determined based on the energy amount to be exerted in performing each energy cycle and a first criterion relating to the target range. The first criterion can represent the target range of the heart rate in terms of a target range of power to be exerted by the subject. The target activity rate can be determined from a power in the target range of power (e.g. a maximum power) divided by the determined energy amount exerted by the subject for each first activity cycle. This means that, for example, an upper limit of the target heart rate range can be represented by an upper limit of the power to be exerted by the subject, and a lower limit of the target heart rate range can be represented by a lower limit of the power to be exerted by the subject. In some cases, the target range of the heart rate of the subject may only be defined by an upper limit on the heart rate.

Then, in step 107, an indication is output to the subject (for example using user interface 10) relating to the determined target activity rate. For example, the indication could be an indication of the target activity rate for repeating the activity cycle (e.g. 1 footstep per second). As another example, the indication could be an indication of a maximum rate at which the subject could repeat the activity cycle in order to keep the heart rate below a maximum permitted heart rate. In another example, the output indication can be a timing indicator corresponding to repetitions of the first activity cycle (e.g. a beep corresponding to footsteps to be taken by the subject).

In some embodiments, the heart rate of the subject can be measured over time (e.g. to produce a time series of heart rate measurements), and these measurements can be analysed to identify changes in a resting heart rate for the subject. The first criterion may be adjusted based on identified changes in the resting heart rate. For example, adjusting the first criterion can include reducing a maximum heart rate of the target range if the resting heart rate increases over time, and adjusting the first criterion according to the reduced maximum heart rate.

In some embodiments, the method can additionally include receiving one or more inputs relating to one or more parameters indicating a current status of the subject (for example parameters such as the weight of the subject, the body composition of the subject, the proportion of oxygen in the air where the subject is located, the environmental temperature and/or humidity, the medication for the subject, the metabolic rate for the subject, and/or an occurrence of arrhythmia), and one or both of the energy amount determined in step 103 and the first criterion can be adjusted based on the received one or more parameters. This adjustment is described in more detail below.

In some embodiments, movement measurements of the subject can be analysed to determine the rate at which the subject is repeating the first activity cycle (this is referred to as the 'first activity rate'). This step can comprise first identifying the activity being performed, and then dividing that activity into activity cycles. The first activity rate can be compared to the target activity rate determined in step 105, and an indication output to the subject based on the result of the comparison. For example, the indication can indicate that the current rate of repeating the first activity cycle should be decreased (for example if it is determined that the current rate at which the subject is repeating the first activity cycle is higher than the target activity rate) or increased (for example if it is determined that the current rate at which the subject is repeating the first activity cycle is lower than the target activity rate).

In some particular embodiments, prior to the apparatus 2 being used, a subject can be required to perform a desired physical activity, for example a physical activity that they are to perform during a regular exercise/training regime, while wearing an energy expenditure meter (e.g. a mask that can be used to measure CO2) in clinical laboratory setting. Each activity cycle (being the smallest repeating motion, e.g. a footstep) of the activity/exercise can be logged in terms of time (e.g. duration of a cycle) and energy consumed. In practice there will usually be an averaging of many cycles to realise an accurate energy consumption per cycle. In daily life, the subject (e.g. a person with heart failure, HF) will use the apparatus 2 (e.g. in the form of a wearable activity detector capable of determining the type of the activity as well as above mentioned activity cycles and their durations). The duration and type of activities performed by the subject will be linked to a library (look-up table/database) of recorded physical activities and energy expenditure in order to estimate the power (i.e. rate of energy expenditure) of the current activity being performed by the subject (step 103). In some embodiments, where the power exerted by the subject is changing over time, e.g. the subject is increasing their running pace, the rate of increase in the repetition rate can be used to extrapolate the power to identify a time at which the subject will be exerting too much power (so their heart rate will be too high), assuming that the rate of increase continues, and/or to identify a maximum rate at which the subject should repeat the activity cycle in order for the subject to keep their heart rate within safe limits (i.e. as set out in step 107 above). By providing an indication to the subject about the target activity rate, the subject is able to adjust their power before they go over the power/heart rate limit. The power boundaries represented by the first criterion may be defined by a care provider on the basis of the severity of the subject's heart failure, and/or the subject's physical mobility/fitness level, in a similar way to current heart rate 'fencing' approaches.

The following relates to techniques for implementing step 101, and for determining/measuring activities during a test/set-up phase in which the energy expended by a subject in performing activity cycles is being measured and then stored for subsequent use in step 103.

Determining the type of activity being performed by a subject from movement measurements is well-known in the art. In particular, techniques for identifying and distinguishing activities such as walking, running, climbing stairs, sitting down, swimming, performing exercises from movement measurements are well known. In some examples, the algorithm used to process the measurements can be based on a supervised machine learning algorithm based on features extracted from movement measurements.

Since for accurate estimation of power exerted the duration of each activity cycle within the activity should be known, the movement measurements need to be analysed to identify the start and/or end point of each activity cycle. This can be implemented using a second layer classifier which would label the start/end point of each cycle. In the case of running, the detection of the start/end of an activity cycle should be straightforward and techniques for detecting a footstep, heel off, etc. are known in the art, for example in devices that count steps. It will be appreciated that an activity cycle may include a single step, but it could include two or more steps.

During the set-up phase, from the determined activity, the type and amount of muscle being used to complete the activity can be derived. Using known techniques, this will enable an amount of energy expenditure to be determined for the activity. The type and amount of muscle being used can be determined using computer simulations of human movement using multiple-segment, multiple-muscle models. Most models use some form of a standard two- or three-element Hill-type muscle model which allow for a straightforward assessment of the mechanical energetics of a subject that is performing an activity. Those skilled in the art will be aware of other types of models that can be used.

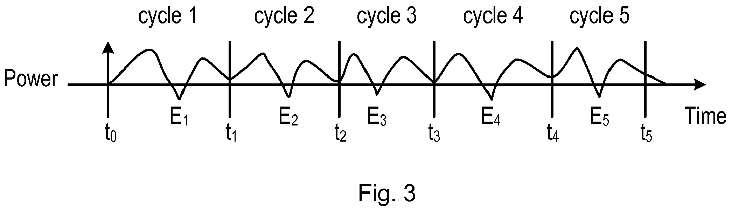

In embodiments of the method, the combination of the energy needed for the activity cycle and the frequency of this activity (the repetition rate) will provide the power exerted by the subject at a given time. Since power scales generally linearly with heart rate, the power exerted provides a surrogate measurement for the heart rate, as shown in Fig. 3.

The graph in Fig. 3 shows exerted power over time while a subject is performing an activity. The activity is a repetition of an activity cycle (e.g. a footstep), and five activity cycles are shown in Fig. 3. The energy expended in each activity cycle is denoted $E_n$ (where n is the number of the cycle, and each activity cycle is between time $t_{n-1}$ and $t_n$), and is given by the integral of the exerted power over time (the duration of the cycle).

One use of the apparatus 2 could be when the subject is at their workplace, and in the morning the apparatus 2 (processing unit 4) can assume (e.g. from an electronic schedule) that the subject needs to get to a particular office or meeting room. This will require some movement and exercise. The apparatus 2 can perform the method in Fig. 2 and indicate to the subject the target activity rate for walking to the office or meeting room. If the distance to be covered is known, the indication provided in step 107 can also or alternatively include how much time the subject can take to achieve the activity (since the power limit provides a maximum repetition rate for the activity cycle, this provides an indication of how quickly the subject can cover that distance).

As another use of the apparatus 2, if the subject is faced with climbing a set of steps, the apparatus 2 will extract the relevant energy expenditure for climbing a step from the look-up-table or database (step 103) and convert this to a safe level of power generation (step 105) by calculating the time range (minimum and maximum time) at which a single step can be climbed (as energy = power x time). The apparatus 2 can then communicate this minimum time to the subject in step 107 using any sort of periodic communication (sound (e.g. beep), tactile (vibration), light, etc.), so that the subject is instructed to climb the steps at the required rate of repetition to avoid exceeding their heart rate limit.

In further embodiments, changes in the baseline heart rate (also referred to as resting heart rate) over time are tracked to determine the subject's level of physical fitness. An elevated resting HR, a shorter HR elevation time, and a slower HR recovery time are indicative of a subject not being in a suitable physical condition to perform physical activities. This can be used to adjust the training regime of the subject to enable the subject to still perform training but in a safer HR range, i.e. the target activity rate for an activity can be reduced if the HR is elevated. For example, the change in baseline HR can be used to modify the criterion used to determine the target activity rate of repetition. Having determined the same limits on the physical activity for the subject, the apparatus 2 can output advice or information to the subject to reassure them that they are physically able to perform the training activity as specified for them.

In another embodiment, potential episodes of arrhythmia such as atrial fibrillation (AF) can be detected, for example from heart rate measurements, and the training level (i.e. rate of repetition of the activity cycle) can be lowered during such episodes.

In another embodiment, the power limits for the subject determined in step 105 may be adjusted to accommodate fluctuations (e.g. daily fluctuations) in the energy expenditure (i.e. the power exerted over time) of subjects during the performance of a given physical activity that utilises specific muscle groups. These variations may arise since the clinical measurements of the energy expended per activity cycle (i.e. that were obtained when the subject was wearing an energy expenditure meter in a clinical laboratory setting) are only acquired at one point in time and may not be fully reproducible on a day-to-day basis, or may not account for changes in a subject's physical status/capabilities over time (e.g. due to changes in body weight, disease progression, etc.) and/or changes in the environment (e.g. changes in temperature, relative humidity, and barometric pressure (due for example to altitude)). Therefore, in this embodiment this problem can be addressed by adjusting the power limits (repetition rate) per exercise or muscle group automatically by a factor, $f$, which ranges between 0 and 1, on the basis of clinically important or subject-specific and environmental parameters affecting the subject's physical status. The parameters can be input manually by the subject or another user of the apparatus 2 (e.g. a care provider), or they can be obtained automatically from another device (or devices) that measures the parameters. The parameters can include, but are not limited to, the following: (i) body weight (weight gain leads to greater energy expenditure per activity cycle while weight loss results in less energy expenditure), (ii) body composition (which can be obtained automatically from a weight scale), with an increasing proportion of fat mass and extracellular fluid volume leading to greater energy expenditure per activity cycle, (iii) altitude (lower barometric pressures increase energy expenditure, e.g. more energy is expended performing an activity at high elevation relative to sea level due to a lower concentration of oxygen), (iv) environmental temperature and humidity, (v) medication administration (e.g. the type of drug and adverse of effects of medications), and (vi) metabolic rate (which is influenced by factors including exercise, stress, fear and illnesses), etc. It should be appreciated that one or more of these parameters may be combined and/or weighted to modulate the power limits of a specific subject. This modulation can be expressed as follows:

$$f = \prod_{i=1}^{N}(p_i),\ 0 < f \le 1 \text{ and } 0 < p \le 1 \qquad (1)$$

where p is a factor linked to a parameter affecting the subject's power per activity and N is the number of parameters affecting the subject's power.

It will be appreciated that the apparatus 2 and method described herein can be used for various purposes. For example, they can be used to support behavioural change of subjects (e.g. exercising more), particularly those with heart failure. It can be used by the subject to manage (limit) the physical load during activities when heart rate or similar measures are difficult to obtain, for example due to arrhythmias. They can also be used to support the recovery of subjects from MI or open heart surgery, who have heart irregularities and need to gradually increase the level of rehabilitation exercise intensity, as prescribed by a cardiologist. Another use can be to support subjects who have drug- and/or food-induced arrhythmias. Examples of arrhythmogenetic medications and foods include Benadryl (e.g. Diphenhydramine), cocaine, caffeine, Tricyclic antidepressants (e.g. Aventyl, Pamelor), sugar, and alcohol, etc.

There is therefore provided an improved way of advising a subject, particularly a subject that has arrhythmia for whom direct heart rate monitoring does not provide reliable measurements.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A computer-implemented method of determining a target activity rate for a subject, the method comprising:

    determining a first activity that is being performed by the subject or that is to be performed by the subject, wherein performing the first activity comprises repeating a first activity cycle;
    determining an energy amount to be exerted by the subject in performing each first activity cycle;
    determining a target activity rate at which the subject should repeat the first activity cycle such that the heart rate of the subject is in a target range, wherein the target activity rate is determined based on the determined energy amount and a first criterion relating to the target range; and
    outputting an indication to the subject relating to the determined target activity rate.

2. A method as defined in claim 1, wherein the step of determining the energy amount comprises:
    retrieving information on the energy amount to be exerted in performing each first activity cycle from a database.

3. A method as defined in claim 1 or 2, wherein the first criterion represents the target range of the heart rate in terms of a target range of power to be exerted by the subject.

4. A method as defined in claim 3, wherein the step of determining the target activity rate comprises:
    determining the target activity rate from a power in the target range of power divided by the determined energy amount exerted by the subject for each first activity cycle.

5. A method as defined in any of claims 1-4, wherein the method further comprises the steps of:

    measuring the heart rate of the subject over time;
    analysing the measurements of the heart rate to identify changes in a resting heart rate for the subject over time; and
    adjusting the first criterion based on identified changes in the resting heart rate.

6. A method as defined in any of claims 1-5, wherein the method further comprises the steps of:

    receiving one or more inputs relating to one or more parameters indicating a current status of the subject; and
    adjusting one or both of (i) the determined energy amount to be exerted by the subject in performing each first activity cycle, (ii) the first criterion based on the received one or more parameters.

7. A method as claimed in any of claims 1-6, wherein the method further comprises:

    determining a first activity rate at which the subject is repeating the first activity cycle;
    comparing the first activity rate to the target activity rate; and
    outputting an indication to the subject based on the result of the comparison.

8. A computer program product comprising a computer readable medium having computer readable code embodied therein, the computer readable code being configured such that, on execution by a suitable computer or processor, the computer or processor is caused to perform the method of any of claims 1-7.

9. An apparatus for managing a heart rate of a subject, the apparatus comprising a processing unit configured to:

    receive information indicative of a first activity that is being performed by the subject or that is to be performed by the subject, wherein performing the first activity comprises repeating a first activity cycle;
    determine an energy amount to be exerted by the subject in performing each first activity cycle;
    determine a target activity rate at which the subject should repeat the first activity cycle such that the heart rate of the subject is in a target range, wherein the target activity rate is determined based on the determined energy amount and a first criterion relating to the target range; and
    output an indication to the subject relating to the determined target activity rate.

10. An apparatus as defined in claim 9, wherein the processing unit is configured to determine the energy amount by retrieving information on the energy amount to be exerted in performing each first activity cycle from a database.

11. An apparatus as defined in claim 9 or 10, wherein the first criterion represents the target range of the heart rate in terms of a target range of power to be exerted by the subject.

12. An apparatus as defined in claim 11, wherein the processing unit is configured to determine the target activity rate by: determining the target activity rate from a power in the target range of power divided by the determined energy amount exerted by the subject for each first activity cycle.

13. An apparatus as defined in any of claims 9-12, wherein the processing unit is further configured to:

   receive measurements of the heart rate of the subject over time;
   analyse the measurements of the heart rate to identify changes in a resting heart rate for the subject over time; and
   adjust the first criterion based on identified changes in the resting heart rate.

14. An apparatus as defined in any of claims 9-13, wherein the processing unit is further configured to:

   receive one or more inputs relating to one or more parameters indicating a current status of the subject; and
   adjust one or both of (i) the determined energy amount to be exerted by the subject in performing each first activity cycle, (ii) the first criterion based on the received one or more parameters.

15. An apparatus as claimed in any of claims 9-14, wherein the processing unit is further configured to:

   determine a first activity rate at which the subject is repeating the first activity cycle;
   compare the first activity rate to the target activity rate; and
   output an indication to the subject based on the result of the comparison.

Fig. 1

101

103

105

107

Fig. 2

Fig. 3

Europäisches
Patentamt
European
Patent Office
Office européen
des brevets

## EUROPEAN SEARCH REPORT

Application Number

EP 18 18 4602

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/216672 A1 (WISBEY BEN [AU] ET AL) 3 August 2017 (2017-08-03) <br> * abstract * <br> * paragraph [0043] - paragraph [0069] * <br> * paragraph [0083] - paragraph [0084] * <br> * paragraph [0090] - paragraph [0107] * <br> * paragraph [0121] * <br> * paragraph [0152] - paragraph [0163] * <br> * claims 1,15 * <br> * figures 1,8B,10A,10B,12B * | 1-15 | INV. <br> G16H20/30 |
| A | US 8 784 115 B1 (CHUANG THOMAS CHU-SHAN [US]) 22 July 2014 (2014-07-22) <br> * the whole document * | 1-15 | |
| A | US 2012/015778 A1 (LEE ALAN RUSSELL [US] ET AL) 19 January 2012 (2012-01-19) <br> * the whole document * | 1-15 | |

TECHNICAL FIELDS
SEARCHED      (IPC)

G16H
G06F
A61B

The present search report has been drawn up for all claims

| Place of search | Date of completion of the search | Examiner |
|---|---|---|
| Munich | 24 January 2019 | Hilbig, Matthias |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 18 4602

This annex lists the patent family members relating to the patent documents cited in the above-mentioned European search report.
The members are as contained in the European Patent Office EDP file on
The European Patent Office is in no way liable for these particulars which are merely given for the purpose of information.

24-01-2019

| Patent document cited in search report | | | Publication date | Patent family member(s) | | | Publication date |
|---|---|---|---|---|---|---|---|
| US 2017216672 | A1 | | 03-08-2017 | NONE | | | |
| US 8784115 | B1 | | 22-07-2014 | NONE | | | |
| US 2012015778 | A1 | | 19-01-2012 | CN | 102339357 | A | 01-02-2012 |
| | | | | CN | 104361207 | A | 18-02-2015 |
| | | | | EP | 2407219 | A2 | 18-01-2012 |
| | | | | JP | 5200142 | B2 | 15-05-2013 |
| | | | | JP | 6066162 | B2 | 25-01-2017 |
| | | | | JP | 2012020134 | A | 02-02-2012 |
| | | | | JP | 2012143601 | A | 02-08-2012 |
| | | | | JP | 2017042620 | A | 02-03-2017 |
| | | | | US | 2012015778 | A1 | 19-01-2012 |
| | | | | US | 2012173978 | A1 | 05-07-2012 |

EPO FORM P0459

For more details about this annex : see Official Journal of the European Patent Office, No. 12/82